# FASCICULE DE BREVET EUROPEEN

(11) **EP 0 659 070 B1**
(45) Date de publication et mention de la délivrance du brevet: **07.05.1997**
(21) Numéro de dépôt: 93919449.4
(22) Date de dépôt: 13.09.1993
(51) Int. Cl.: A61K 7/48, A61K 7/00, C09B 67/00, C09C 3/10

(54) **COMPOSITION COSMETIQUE CONTENANT DES PARTICULES SOLIDES REVETUES AVEC UN POLYMERE AMPHOTERE**
KOSMETISCHE ZUSAMMENSETZUNG, DIE FESTE, MIT EINEM AMPHOTEREN POLYMER ÜBERZOGENE TEILCHEN ENTHÄLT
COSMETIC COMPOSITION CONTAINING SOLID PARTICLES COATED WITH AN AMPHOTERIC POLYMER

(30) Priorité: 11.09.1992 FR 9210884
(43) Date de publication de la demande: 28.06.1995
(73) Titulaire: L'OREAL, 75008 Paris (FR)
(72) Inventeur: MARCHI, Patricia, F-75008 Paris (FR); MELLUL, Myriam, F-94240 L'Hay-les-Roses (FR)
(74) Mandataire: Tonnellier, Jean-Claude
(86) Numéro de dépôt international: FR9300879
(87) Numéro de publication internationale: WO9406407

(56) Documents cités:
- EP-A- 0 209 879
- EP-A- 0 212 870
- EP-A- 0 220 617
- FR-A- 2 234 359
- FR-A- 2 339 656
- GB-A- 2 107 186
- LU-A- 57 903

## Description

La présente invention a pour objet des compositions cosmétiques pour la peau ou les phanères, contenant une dispersion de particules solides, dont la surface est revêtue à l'aide d'un polymère amphotère.

On sait que divers produits de maquillage tels que les poudres libres ou compactées, les fonds de teint, les fards à joues, les fards à paupières, ainsi que les rouges à lèvres sont présentés sous la forme de compositions comprenant une dispersion de particules minérales solides dans un liant gras. Il peut s'agir de compositions anhydres, ou bien d'émulsions huile-dans-l'eau ou eau-dans-l'huile.

Selon les types de compositions, les particules solides sont uniquement des pigments (blancs et/ou colorés), destinés à conférer à la peau du visage ou des lèvres une certaine coloration, soit des particules, généralement appelées "charges", qui ont des fonctions diverses variant avec la nature des particules.

Dans les compositions à appliquer sur la peau, on utilise souvent des charges destinées à apporter un pouvoir couvrant, c'est-à-dire à masquer les imperfections de la peau (différences de coloration, microreliefs) soit grâce à leur opacité (c'est le cas notamment pour les oxydes de titane et de zinc et pour le kaolin) soit par leurs propriétés de réflexion de la lumière (c'est le cas notamment des charges lamellaires telles que le talc et les micas). On utilise également des charges qui sont capables d'absorber les sécrétions aqueuses et huileuses de la peau, afin d'éviter l'aspect luisant de la peau et la migration des matières colorantes : on emploie par exemple à cet effet le kaolin, l'amidon, le carbonate de calcium précipité, la bentonite, etc...

On utilise aussi, dans des compositions destinées à la protection contre le rayonnement UV, des particules micronisées ou non de TiO₂ et de ZnO comme agents d'absorption de l'ultraviolet.

Dans les rouges à lèvres, les particules solides dispersées dans un liant gras approprié sont surtout des pigments colorés, éventuellement en association avec des pigments blancs (par exemple de fines particules de dioxyde de titane) qui permettent de nuancer les teintes apportées par les pigments colorés.

On utilise également de tels pigments blancs et/ou colorés dans les compositions de vernis à ongles, qui sont essentiellement constituées d'une dispersion de ces pigments dans une solution d'un polymère filmogène et d'un plastifiant dans un solvant organique approprié.

La préparation et l'utilisation des compositions cosmétiques contenant des dispersions de particules solides posent plusieurs sortes de problèmes. Un problème commun à la réalisation de l'ensemble des compositions dont on vient de parler réside dans la difficulté d'obtenir des dispersions homogènes et stables, de façon à appliquer, par exemple sur la peau, un maquillage régulier dont l'application est uniforme et qui conserve une bonne homogénéité. Pour cela, les spécialistes ont été amenés à effectuer des traitements de surface sur les poudres utilisées, notamment afin de modifier les propriétés interfaciales intervenant dans les phénomènes de mouillage et de dispersion. Le but de ces traitements est souvent de rendre la poudre hydrophobe afin de favoriser son incorporation dans les liants et les huiles de formulation, et d'augmenter la stabilité de la dispersion en diminuant les phénomènes de floculation et d'agrégation ; voir par exemple le brevet européen 279 319 qui décrit l'enrobage des pigments par des polymères siliconés.

La dispersion des particules solides dans les milieux aqueux soulève également des difficultés, et pour y remédier, on utilise notamment des agents dispersants qui sont introduits dans le milieu de dispersion. Ainsi, par exemple, le brevet FR 2655875 concerne l'utilisation de copolymères amphotères comme agents de dispersion, en particulier dans le domaine papetier, et le brevet FR 2550795 décrit une composition pigmentaire pour peintures contenant une résine dispersante amphotère. Ces divers traitements permettent donc de régler les problèmes de stabilité de la dispersion, en limitant les phénomènes de floculation. Toutefois, ils ne règlent pas un autre problème important, à savoir les faibles propriétés d'adhérence sur la peau des particules solides. En effet, on sait que les particules solides utilisées notamment dans les compositions sous forme de poudres, n'ont que de faibles propriétés d'adhérence sur la peau. Les traitements de surface destinés à améliorer la stabilité des dispersion dans les liants gras n'apportent pas d'amélioration sensible en ce qui concerne les propriétés d'adhérence.

On a maintenant découvert qu'il est possible d'obtenir des compositions cosmétiques, comprenant une dispersion de particules solides dans un liant, ayant de bonnes propriétés de stabilité et d'adhérence sur la peau ou sur les phanères, en introduisant dans lesdites compositions des particules solides dont la surface a été revêtue avec un polymère amphotère. On a constaté, de façon surprenante, que le revêtement des particules solides par des polymères amphotères qui constituent pourtant un revêtement hydrophile, n'empêche pas l'obtention d'une bonne dispersibilité des particules dans les liants gras. Par ailleurs, les compositions ainsi obtenues ont de bonnes propriétés d'adhérence sur la peau après application. L'adhérence sur la peau n'est pas excessive et permet donc une répartition sur la peau et un résultat de maquillage particulièrement homogènes.

En outre, le revêtement des particules solides avec un polymère amphotère est compatible avec l'utilisation de polymères cationiques dans les compositions cosmétiques contenant de telles particules revêtues.

Par ailleurs, les particules revêtues selon l'invention conservent leur caractère amphotère même après qu'elles sont appliquées sur la peau, et quelle que soit la nature de la particule ainsi revêtue.

Le rapport quantitatif optimal groupements cationiques/groupements anioniques, s'échelonne de 40/60 à 90/10.

Les documents LU-A-57903 et EP-A-212 870 décrivent des compositions cosmétiques contenant des particules solides enrobées de polymères qui sont différents des polymères amphotères tels que définis ci-après.

La présente invention a donc pour objet une composition cosmétique pour la peau ou les phanères, comprenant une dispersion de particules solides dans un liant, caractérisée par le fait qu'au moins une partie desdites particules sont introduites dans ladite composition sous la forme de particules dont la surface est revêtue d'au moins un polymère amphotère.

Dans les compositions de l'invention, des particules solides sont revêtues en surface avec un polymère amphotère. Cela signifie qu'après revêtement, il n'y a ni changement de morphologie ni modification notable des tailles des particules, comme on peut le vérifier par microscopie électronique.

Dans la présente demande, l'expression "polymère amphotère" désigne un polymère contenant à la fois des groupements cationiques et anioniques et/ou des groupements ionisables respectivement en groupements cationiques et anioniques.

Les polymères amphotères sont des produits connus.

Les groupements cationiques préférés sont choisis parmi ceux qui contiennent des groupements amine primaires, secondaires, tertiaires et/ou quaternaires pouvant soit faire partie de la chaîne polymère soit être portés par un substituant latéral.

Le groupement cationique est choisi de préférence parmi les groupements aminés primaires, secondaires, tertiaires et/ou quaternaires. Les groupements anioniques peuvent par exemple être les groupements COO⁻, SO₃⁻, PO₄³⁻, SO₄²⁻.

Ces deux types de groupements chargés (anioniques et cationiques) sont de préférence portés chacun par un substituant latéral et plus ou moins éloignés l'un de l'autre.

De préférence, les groupements cationiques sont des groupements ammonium quaternaires, qui peuvent être obtenus par quaternisation de groupements aminés à l'aide d'agents de quaternisation classiques tels que des halogénures d'alkyle ou d'aralkyle (par exemple iodure de méthyle, bromure d'éthyle, chlorure de benzyle, etc...) ou des sulfates d'alkyle (par exemple le sulfate de diméthyle).

Les polymères amphotères utilisés peuvent avoir une masse moléculaire comprise généralement entre 10³ et 10⁹ environ, le plus souvent entre 10³ et 10⁶.

De préférence, les particules revêtues utilisées dans les compositions de l'invention sont revêtues uniquement avec un (ou plusieurs) polymère(s) amphotère(s).

Lorsque le polymère amphotère porte des groupements cationiques et anioniques sur des substituants latéraux, la chaîne polymère est par exemple une chaîne acrylique, vinylique, siliconée, fluorée ou saccharidique. De préférence, on utilise des polymères amphotères ne contenant pas de silicium, c'est-à-dire des polymères autres que des silicones.

Les quantités de polymère déposées sur les particules varient avec le mode opératoire utilisé pour obtenir le revêtement. Généralement, la proportion pondérale de polymère amphotère par rapport au poids total des particules revêtues, est au moins égale à 0,1% ; la limite supérieure de la quantité de polymère amphotère est suffisamment faible pour que les particules gardent leur individualité et leur forme. Autrement dit, le polymère amphotère forme, au plus, une couche mince (éventuellement lacunaire) sur les particules revêtues. Le plus souvent, la proportion pondérale de polymère amphotère, dans les particules revêtues, est inférieure à 10%, et de préférence inférieure à 8%, par rapport au poids total des particules revêtues. En fait, le plus souvent, on obtient un résultat optimal avec un revêtement ne dépassant pas 2% en poids. On évite ainsi l'obtention de pigments enrobés dont la couleur risque de devenir terne après revêtement.

Les compositions de l'invention sont notamment celles qui contiennent une dispersion de particules solides dont la surface est revêtue d'au moins un polymère amphotère comportant des motifs A et B répartis statistiquement dans la chaîne polymère, où A désigne un motif dérivant d'un monomère comportant au moins un groupement cationique tel que défini précédemment, et B désigne un motif dérivant d'un monomère comportant un ou plusieurs groupements anioniques, notamment carboxyliques ou sulfoniques, ou bien A et B identiques ou différents, représentent un groupement dérivant d'un monomère zwitterionique de carboxybêtaïne ; A et B peuvent également designer une chaîne polymère comportant des groupements amine secondaire, tertiaire ou quaternaire, dans laquelle au moins l'un des groupements amine porte un substituant carboxylique ou sulfonique relié par l'intermédiaire d'un bras hydrocarboné, ou bien A et B font partie d'une chaîne polymère à motifs éthylène alpha, bêta-dicarboxylique dont l'un des groupements carboxyliques a réagi avec une polyamine comportant un ou plusieurs groupements amine primaire ou secondaire.

Les polymères amphotères répondant à la définition indiquée ci-dessus sont choisis notamment parmi les polymères suivants :
(1) les polymères résultant de la copolymérisation d'un monomère dérivé d'un composé vinylique portant un groupement carboxylique tel que l'acide acrylique, l'acide méthacrylique, l'acide maléique, l'acide alpha-chloracrylique, et d'un monomère basique dérivé d'un composé vinylique substitué contenant au moins un atome d'azote basique tel que les dialkylaminoalkyl- méthacrylates et acrylates, et les dialkylaminoalkyl- méthacrylamides et acrylamides. De tels composés sont décrits dans le brevet américain n° 3.836.537.
(2) les polymères comportant des motifs dérivant :
   a) d'au moins un monomère choisi parmi les acrylamides ou les méthacrylamides substitués à l'azote par un radical alkyle,
   b) d'au moins un comonomère acide contenant un ou plusieurs groupements carboxyliques réactifs, et
   c) d'au moins un comonomère basique tel un ester à substituant(s) amine primaire, secondaire, tertiaire et quaternaire des acides acrylique et méthacrylique, ou le produit de quaternisation du méthacrylate de diméthylaminoéthyle avec le sulfate de diméthyle ou de diéthyle.

   Les monomères acrylamide ou méthacrylamide N-substitués plus particulièrement préférés, pour les polymères mentionnés ci-dessus, sont notamment ceux dont les groupements alkyle contiennent de 2 à 12 atomes de carbone, et plus particulièrement le N-éthylacrylamide, le N-tertiobutyl acrylamide, le N-tertio-octyl acrylamide, le N-octylacrylamide, le N-décylacrylamide, le N-dodécylacrylamide, ou les méthacrylamides correspondants. Les comonomères acides sont choisis plus particulièrement parmi les acides acrylique, méthacrylique, crotonique, itaconique, maléique, fumarique ainsi que les monoesters de C₁-C₄ alkyle de l'acide maléique ou de l'acide fumarique.
   Les comonomères basiques sont par exemple des méthacrylates d'aminoéthyle, de butylaminoéthyle, de N,N-diméthylaminoéthyle ou de N-tertio-butylaminoéthyle.
   Parmi ces copolymères, on peut citer les produits vendus par la Société National Starch sous la dénomination Amphomer.
(3) les polyamino amides éventuellement réticulés et/ou alcoylés, partiellement ou totalement, dérivant de polyaminoamides comportant des motifs de formule générale :

   -[-OC-R-CO-Z-]- (I)

   dans laquelle R représente un radical divalent dérivé d'un acide dicarboxylique saturé, d'un acide aliphatique mono- ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalcoylène-polyamine bis-primaire, mono- ou bis-secondaire. En particulier, dans lesdits copolymères, Z peut représenter :
   a) dans une proportion de 60 à 100 moles %, par rapport à l'ensemble des motifs contenant Z, le radical

      -NH-[-CH₂)ₓ-NH-]-ₙ (II)

      où x = 2 et n = 2 ou 3 ou bien x = 3 et n = 2
      (ce radical dérivant donc de la diéthylènetriamine, de la triéthylènetétramine ou de la dipropylènetriamine),
   b) dans une proportion de 0 à 40 moles % le radical (II)) ci-dessus, dans lequel x = 2 et n = 1 (dérivant donc de l'éthylènediamine)
      ou le radical 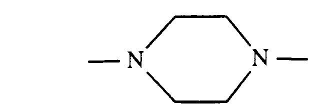 (dérivant de la pipérazine),
   c) dans une proportion de 0 à 20 moles %, le radical -NH-(CH₂)₆-NH- (dérivant de l'hexaméthylènediamine),
   ces (polyamino-amides) étant éventuellement réticulés par addition d'un agent réticulant bifonctionnel choisi parmi les épihalohydrines, les diépoxydes, les dianhydrides et les dérivés bis insaturés, par exemple au moyen de 0,025 à 0,35 mole d'agent réticulant par groupement amine du polyaminoamide, et/ou pouvant être alcoylés par l'action d'acide acrylique, d'acide chloracétique, d'une alcane sultone ou de leurs sels.
   Les acides carboxyliques saturés sont choisis de préférence parmi les acides ayant 6 à 10 atomes de carbone tels que les acides adipique, triméthyl-2,2,4- et -2,4,4-adipiques, l'acide téréphtalique et les acides à double liaison éthylénique comme par exemple les acides acrylique, méthacrylique ou itaconique.
   Les alcane sultones pouvant être utilisées dans l'alcoylation des polyamino-amides. sont par exemple la propane- ou la butane- sultone, les sels desdits agents d'alcoylation étant notamment les sels de sodium ou de potassium.
(4) Les polymères comportant des motifs Zwitterionique dérivés d'un monomère de formule : 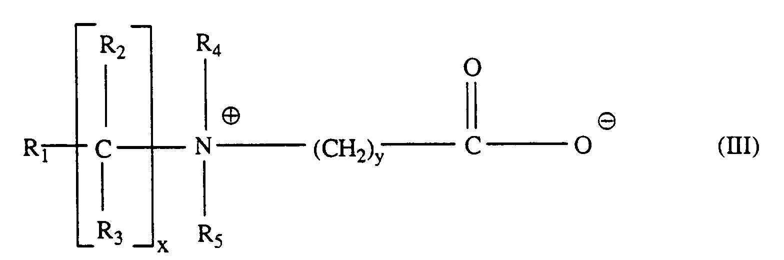 dans laquelle
   R₁ désigne un groupement insaturé polymérisable tel qu'un groupement acrylate, méthacrylate, acrylamide ou méthacrylamide,
   x et y représentent indépendamment un nombre entier de 1 à 3,
   R₂ et R₃ représentant hydrogène, méthyle, éthyle ou propyle,
   R₄ et R₅ représentant un atome d'hydrogène ou un radical alkyle, R₄ et R₅ étant tels que la somme des atomes de carbone qu'ils contiennent ne dépasse pas 10.

   Les polymères comprenant de telles unités peuvent également comporter en outre des motifs dérivés de monomères non zwitterioniques tels que la vinylpyrrolidone, l'acrylate ou le méthacrylate de diméthyl- ou diéthyl- aminoéthyle, ou les acrylates ou méthacrylates d'alkyle, l'acrylamide, le méthacrylamide, ou l'acétate de vinyle.
   Parmi les polymères on peut citer ceux contenant des motifs dérivant de bétaïnes carboxyliques et notamment :
   les copolymères de méthacrylate de méthyle/méthacrylate d'éthyl diméthyl carboxyméthyl ammonium, tels que les produits vendus par Chimex sous la dénomination Mexomere® PX (Nom CTPA ; "*polyquaternium-30"*) de formule : 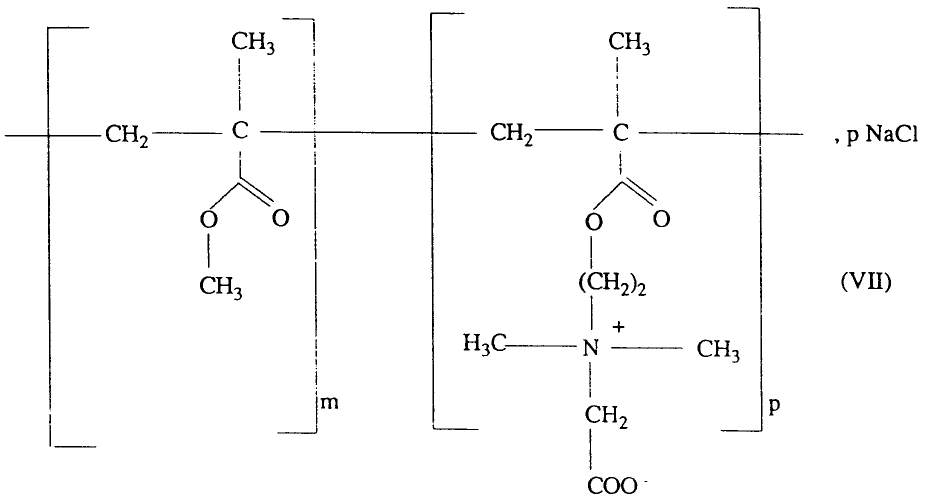
   m ≃ 60
   p ≃ 40,
   ou des polymères analogues contenant des proportions différentes desdits motifs,
   le copolymère méthacryloyléthylbétaïne/méthacrylate vendu par Sandoz sous la dénomination Diaformer®,
   ou le copolymère de méthacrylol éthyl bétaïne/méthacrylate vendu par Amerchol sous la dénomination Amersette®.
   Parmi les polymères amphotères, on peut encore citer les bétaïnes polysiloxanes, tels que les copolymères de polysiloxanes polyorganobétaïnes vendus par Goldschmidt sous la dénomination Abil B 9950® (Nom CTFA "*Diméthicone PropylPG-Bétaïne"*) de formule : 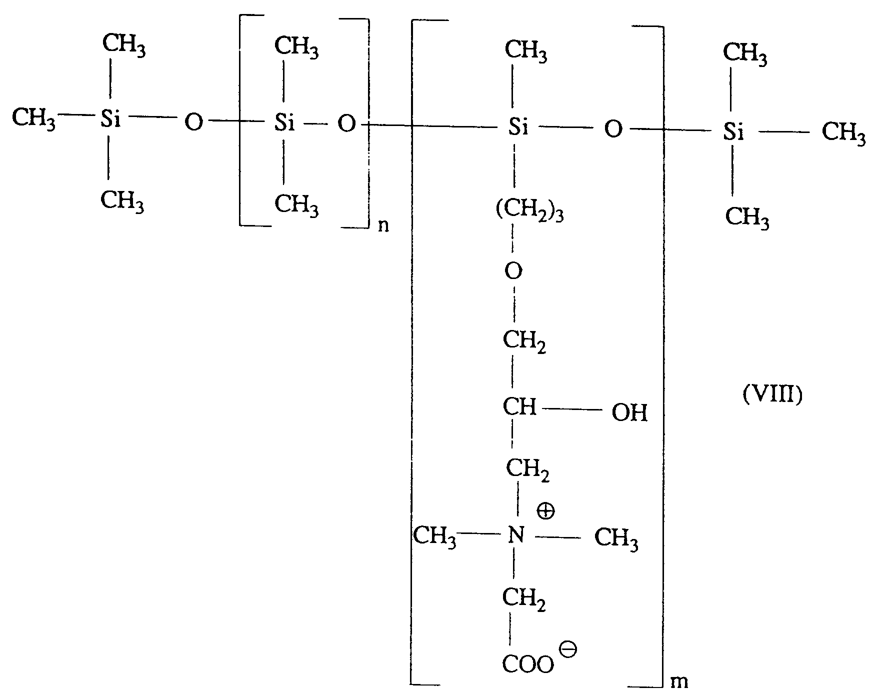 ou le polydiméthylsiloxane à groupements alkyl phosphobétaïne vendu par Siltech sous la dénomination Pecosil® SPB-1240,
   ou le copolymère de siloxane/organobétaïne oxyéthylèneoxypropylène vendu par Goldchmidt sous la dénomination BC 1610.
(5) Les polymères dérivés du chitosane comportant des motifs monomères répondant aux formules suivantes : 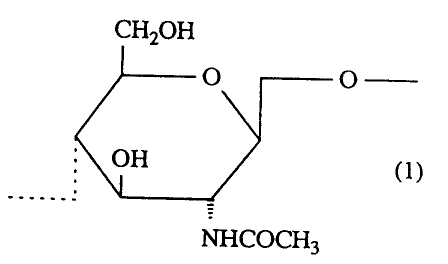 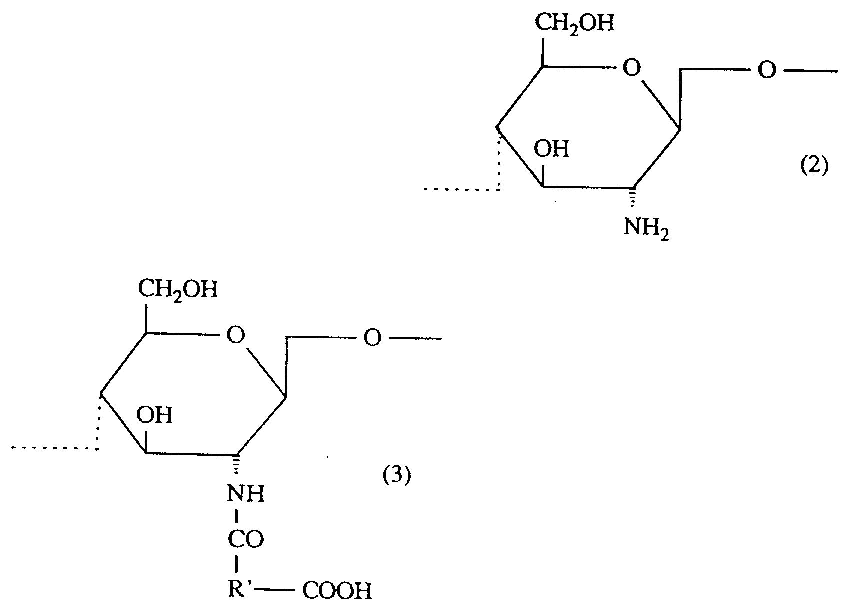 dans lesquels les motifs (1) sont présents dans des proportions comprises entre 0 et 30%, les motifs (2) dans des proportions comprises entre 5 et 50%, et les motifs (3) dans des proportions comprises entre 30 et 90%. Dans la formule (3), R' représente un radical de formule:

   -CR₆(R₇) - (O)ₙ - CH(R₈)-

   dans laquelle
   n est un nombre égal à zéro ou 1,
   si n = 0, R₆, R₇ et R₈ représentent chacun indépendament un atome d'hydrogène, un reste méthyle, hydroxyle, acétoxy, amino, monoalcoylamine ou dialcoylamine (éventuellement interrompu par un ou plusieurs atomes d'azote et/ou éventuellement substitué par un ou plusieurs groupes amine, hydroxyle, carboxyle, alcoylthio ou sulfonique) ou un reste alcoylthio dont le groupe alcoyle porte un substituant amino, l'un au moins des radicaux R₆, R₇ et R₈ étant dans ce cas un atome d'hydrogène,
   ou n est égal à 1, auquel cas R₆, R₇ et R₈ représentent chacun un atome d'hydrogène,
   ainsi que les sels formés par ces composés avec des bases ou des acides.
   Parmi ces polymères on peut citer les polymères dérivés du chitosane par succinylation d'un certain pourcentage des groupes animés tels que les produits vendus par Chimex sous la dénomination Mexomere® PAD (Nom CTFA "*Chitosan Succinamide"*).
(6) Les polymères contenant des motifs de formule générale (IV) : 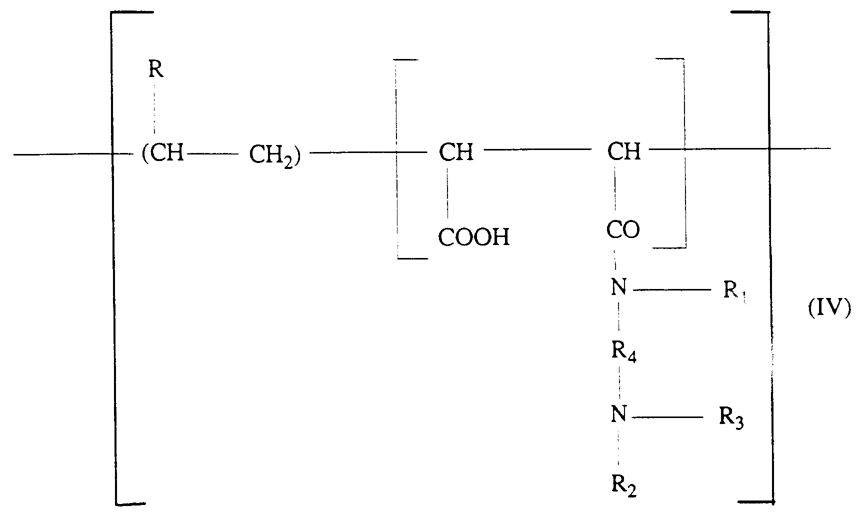 dans laquelle R représente un atome d'hydrogène ou un radical CH₃O-, CH₃CH₂O- ou phényle, R₁ et R₂ représentent indépendamment un hydrogène ou un radical alcoyle inférieur tel que méthyle ou éthyle, et R₃ désigne un radical alcoyle inférieur tel que méthyle ou éthyle, ou un groupement -R₄-N(R₂)₂, R₄ représentant un groupement alkylène contenant de 2 à 6 atomes de carbone, et R₂ étant défini comme précédemment.
   De tels polymères sont décrits dans le brevet français 1 400 366.
(7) Des polymères amphotères choisis parmi :
   - les polymères obtenus par l'action de l'acide chloracétique ou du chloracétate de sodium sur les composés comportant au moins un motif de formule

      -A-Z-A-Z-A- (V)

      où A désigne un radical 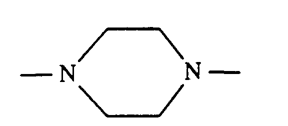 et les groupements Z représentant indépendamment un radical alkylène comportant jusqu'à 7 atomes de carbone dans la chaîne principale, éventuellement substitué par un ou plusieurs groupements hydroxyle et pouvant comporter en outre des atomes d'oxygène, d'azote, de soufre et/ou 1 à 3 cycles aromatiques et/ou hétérocycliques, lesdits atomes d'oxygène, d'azote et de soufre pouvant être présents sous forme de groupement éther, thioéther, sulfoxyde, sulfone, sulfonium, alkylamine, alkénylamine, hydroxyle, benzylamine, oxyde d'amine, ammonium quaternaire, amide, imide, alcool, ester et/ou uréthane ;
   - les polymères contenant des motifs de formule

      -A'-Z-A'-Z- (VI)

      où A' désigne un radical 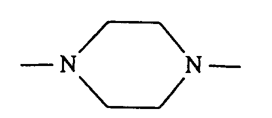 et où l'un au moins des groupements Z est tel que défini ci-dessus, et l'un au moins des groupements Z représente un radical alkylène ayant jusqu'à 7 atomes de carbone dans la chaîne principale, éventuellement substitué par un ou plusieurs radicaux hydroxyle et comportant un ou plusieurs atomes d'azote substitués par une chaîne alkyle interrompue éventuellement par un atome d'oxygène, et comportant obligatoirement une ou plusieurs fonctions hydroxyle et/ou carboxyle, ainsi que les sels d'ammonium quaternaires résultant de la réaction de l'acide chloracétique ou du chloracétate de sodium sur les polymères (V).

Parmi les copolymères acryliques et/ou méthacryliques amphotères, on peut citer également les copolymères de chlorure d'ammonium et d'acide acrylique tels que le produit vendu par Calgon sous la dénomination Polyquaternum-22®, le copolymère acrylique séquencé vendu par Kingston sous la dénomination Hypan SS 430E® ou le copolymère d'acide acrylique/chlorure de diméthyl diallyl ammonium/acrylamide vendu par Merck sous la dénomination Merquat® Plus 3330, ou encore le copolymère de chlorure de diméthyl diallyl ammonium et d'acide acrylique vendu par Merck sous la dénomination Merquat® 280.

Les particules revêtues présentes dans la composition de l'invention sont notamment des charges ou des pigments minéraux, ou encore des particules organiques.

Les charges minérales, naturelles ou synthétiques sont par exemple choisies parmi : les carbonates de calcium, les silicates comme par exemple le silicate d'aluminium ou kaolin, les silicates de calcium, l'alumino-silicate de sodium, le silicate de magnésium ou talc, les alumino-silicates de potassium ou micas et l'alumin-silicate de magnésium hydraté; les sulfates comme par exemple le sulfate de baryum, le sulfate de calcium ; les dioxydes de silicium précipités ou pyrogénés, ainsi que les hydrogels et aérogels de silice.

Les pigments sont choisis par exemple parmi les pigments blancs tels que le dioxyde de titane ou l'oxyde de zinc et les pigments colorés tels que : des oxydes de fer colorés (naturels ou synthétiques) de couleurs noire, rouge et jaune; des oxydes de chrome verts hydratés ou nom ; le bleu de Prusse; les alumino-sulfosilicates de sodium et leur différentes variantes connues sous le nom de pigments "ultramarines" ; l'aluminate de cobalt ou bleu de cobalt et le violet de manganèse.

Les pigments peuvent être encore :
- soit des pigments nacrés tels que les mica-titanes (mica revêtu de particules de dioxyde de titane) et l'oxychlorure de bismuth ;
- soit des pigments micronisés d'oxydes métalliques choisis parmi les oxydes de titane, de zinc, de cérium, de zirconium, ou leurs mélanges.

Les pigments destinés à être revêtus conformément à l'invention peuvent être éventuellement des pigments ayant subi préalablement un ou plusieurs traitements de surface de nature chimique, électronique et/ou mécanique. Il peuvent également être des pigments composites à revêtement organiques tels que ceux qui sont décrits ci-après.

Les particules organiques destinées à être revêtues par un polymère amphotère, selon l'invention, comprennent par exemple :
- le carmin de cochenille,
- le noir de carbone,
- les laques organiques ou sels insolubles de sodium, de potassium, de calcium, de baryum, d'aluminium, de zirconium ou de strontium, de colorants acides tels que les colorants halogénoacides, azoïques, anthraquinoniques, etc... Parmi ces laques, on peut en particulier citer celles connues sous les dénominations suivantes : 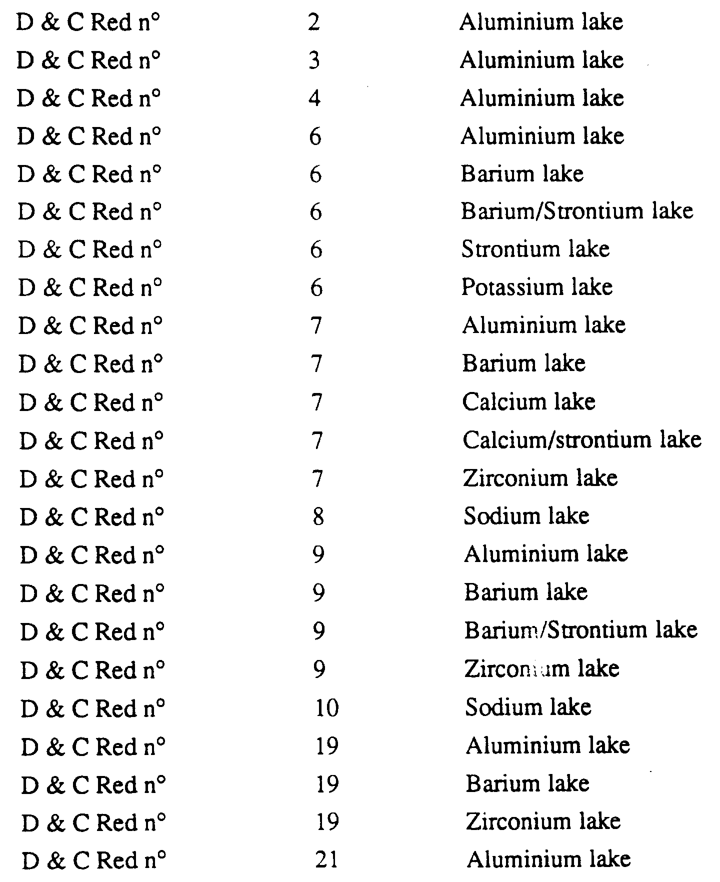 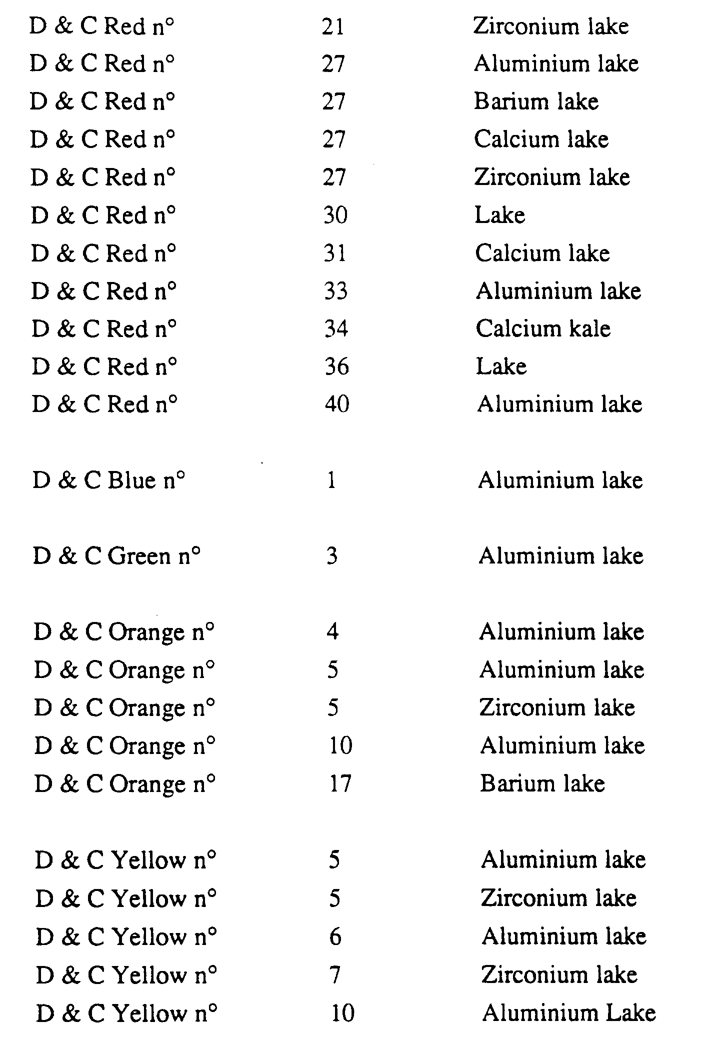
- les pigments mélaniques dérivés de sources naturelles ou synthétiques et qui peuvent être obtenus : (A) par oxydation d'au moins un composé indolique, ou (B) par polymérisation oxydante ou enzymatique de précurseurs mélaniques, ou (C) par extraction de la mélanine à partir de substances en contenant, ou (D) par culture de microorganismes.

(A) Les pigments mélaniques peuvent, en premier lieu, être obtenus par oxydation d'au moins un composé indolique choisi notamment parmi ceux répondant à la formule : 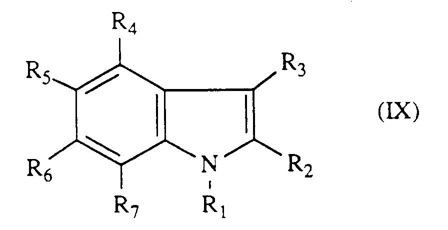 dans laquelle :
   - R₁ et R₃ représentent, indépendamment l'un de l'autre, un atome d'hydrogène ou un groupe alkyle en C₁-C₄ ;
   - R₂ représente un atome d'hydrogène, un groupe alkyle en C₁-C₄, un groupe carboxyle ou un groupe alcoxy (C₁-C₄)-carbonyle;
   - les substituants R₄ à R₇ représentent un atome d'hydrogène, un groupement alkyle en C₁-C₄, un groupement -NHR° ou -OZ,
      R° désignant un atome d'hydrogène, un groupe acyle en C₂-C₄ ou hydroxyalkyle en C₂-C₄, et le radical Z désignant un atome d'hydrogène, un groupe acyle en C₂-C₁₄, un groupe alkyle en C₁-C₄, ou un groupe triméthylsilyle,
   - étant entendu que R₅ peut en outre représenter un atome d'halogène, et étant entendu que :
   - au moins l'un des radicaux R₄ à R₇ représente un groupement OZ ou NHR°, l'un au plus des radicaux R₄ à R₇ représentant NHR° et deux au plus des radicaux R₄ à R₇ représentant OZ et, dans le cas où Z représente un atome d'hydrogène, les deux groupes OH sont dans les positions 5 et 6 ; et au moins l'un des radicaux R₄ à R₇ représente un atome d'hydrogène, et dans le cas où un seul de ces radicaux représente un atome d'hydrogène, un seul radical parmi les radicaux R₄ à R₇ représente alors NHR° ou OZ, les autres radicaux représentant un groupe alkyle en C₁-C₄, ou encore, le cas échéant, pour R₅, un atome d'halogène ;
   et leurs sels de métaux alcalins, alcalino-terreux, d'ammonium, d'amines, ainsi que les chlorhydrates, les bromhydrate, les sulfates et méthanesulfonates.
   Les composés indoliques de formule (IX) ci-dessus sont choisis, de préférence, parmi le 4-hydroxyindole, le 5-hydroxyindole, le 6-hydroxyindole, le 7-hydroxyindole, le 4-hydroxy-5-méthoxyindole, le 4-hydroxy-5-éthoxyindole, le 2-carboxy-5-hydroxyindole, le 5-hydroxy-6-méthoxyindole,le 6-hydroxy-7-méthoxyindole, le 5-méthoxy-6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthyl indole, le 2-carboxy 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5 méthoxy 2,3-diméthylindole, le 6-hydroxy 2-éthoxycarbonylindole, le 7-β-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N-β-hydroxyéthylaminoindole, le 4-aminoindole, le 5-aminoindole, le 6-aminoindole, le 7-aminoindole, le N-méthyl 6-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6-diacétoxyindole, le 5-méthoxy 6-acétoxyindole, le 5,6-diméthoxyindole, le 5,6-méthylènedioxyindole, le 5,6-triméthylsilyloxyindole, l'ester phosphorique du 5,6-dihydroxyindole, le 5,6-dibenzyloxyindole, et les sels d'addition de ces composés.
   Le 5,6-dihydroxyindole est l'un des composés préférés.
   L'oxydation du composé indolique de formule (IX) peut s'effectuer en milieu aqueux ou eau-solvant(s), à l'air, en présence ou non d'un agent alcalin et/ou d'un catalyseur métallique d'oxydation tel que par exemple l'ion cuivrique.
   Le milieu réactionnel est, de préférence, constitué par de l'eau et peut, le cas échéant, être constitué par un mélange d'eau et d'au moins un solvant choisi de telle façon qu'il solubilise rapidement le composé indolique de formule (IX). Parmi ces solvants, on peut citer, à titre d'exemples, les alcools inférieurs en C₁-C₄, tels que l'alcool éthylique, l'alcool propylique ou isopropylique, l'alcool tert-butylique, les alkylèneglycols tels que l'éthylèneglycol, le propylèneglycol, les alkyléthers d'alkylèneglycols, tels que les éthers monométhylique, monoéthylique et monobutylique de l'éthylèneglycol, les monométhyléthers du propylèneglycol et du dipropylèneglycol, et le lactate de méthyle.
   L'oxydation peut également s'effectuer en mettant en oeuvre le peroxyde d'hydrogène en présence d'un agent alcalin, tel que, de préférence, l'ammoniaque, ou en présence d'un ion iodure, l'iodure étant, de préférence, l'iodure d'un métal alcalin, alcalino-terreux ou d'ammonium.
   On peut également procéder à l'oxydation en utilisant l'acide periodique et ses sels hydrosolubles et dérivés, les permanganates et bichromates, par exemple de sodium ou de potassium, l'hypochlorite de sodium, le ferricyanure de potassium, le persulfate d'ammonium, l'oxyde d'argent, l'oxyde de plomb, le chlorure ferrique, le nitrite de sodium, les sels de terres rares dont, notamment le cérium, et des oxydants organiques choisis parmi les ortho- et parabenzoquinones, les ortho- et parabenzoquinones mono- ou di-imines, les 1,2- et 1,4-naphtoquinones, les 1,2- et 1,4-naphtoquinones mono- ou di-imines telles que définies dans la demande EP-A-0 376 776. Le sel d'acide periodique préféré est le periodate de sodium.
   Il est possible d'activer les agents oxydants par un modificateur de pH.
   On peut également effectuer une oxydation enzymatique.
   Le produit insoluble est isolé par filtration, centrifugation, lyophilisation ou atomisation ; il est ensuite broyé ou micronisé pour atteindre la granulométrie désirée.
(B) Les pigments mélaniques peuvent également provenir de la polymérisation oxydante ou enzymatique de précurseurs mélaniques, tels que la L-tyrosine, la L-dopa, le catéchol et leurs dérivés.
(C) les pigments mélaniques peuvent enfin provenir de l'extraction de la mélanine de substances naturelles telles que les cheveux humains ou l'encre de céphalopodes (seiches, poulpes), encore connue sous le nom de sépiomélanine, auquel cas le pigment est broyé et purifié avant sont utilisation.
(D) Les pigments mélaniques peuvent être obtenus par culture de microorganismes. Ces microorganismes produisent de la mélanine soit naturellement, soit par modification génétique ou par mutagénèse. Des modes de préparations de ces mélanines sont décrits par exemple dans la demande de brevet WO-90 04029.

Le pigment mèlanique peut être présent à la surface, ou incorporé dans une charge particulaire, minérale ou organique, lamellaire ou non lamellaire, colorée ou non. On obtient ainsi des pigments mélaniques composites.

Dans ce cas, le pigment mélanique peut résulter de l'oxydation d'au moins un composé indolique de formule (IX), telle que définie ci-dessus, en mélange avec la charge particulaire, dans un milieu essentiellement non-solvant pour ladite charge, à une température pouvant aller de la température ambiante jusqu'à environ 100°C, ou encore résulter de la polymérisation oxydante de précurseur mélanique sur la charge.

Les particules minérales non lamellaires utilisées dans ce procédé sont en particulier des particules minérales inertes ayant une granulométrie inférieure à 20 micromètres. De telles particules sont notamment les particules de carbonate de calcium, de silice ou d'oxyde de titane.

De tels pigments mélaniques composites, déposés sur charges minérales, sont décrits, ainsi que leur préparation, dans la demande de brevet FR-2.618.069.

Par un procédé analogue, on peut préparer des pigments mélaniques composites avec des particules minérales colorées.

On appelle "particules minérales colorées" des particules non blanches constituées de sels métalliques, insolubles dans le milieu cosmétique, utilisables en cosmétique telles que celles référencées dans le Color Index sous le chapitre "Inorganic Colouring Matters" et portant les numéros 77000 à 77947, à l'exclusion des pigments blancs et de particules se présentant sous forme lamellaire telles que l'oxyde de fer lamellaire. Ces particules minérales colorées peuvent être constituées d'un seul pigment ou d'un mélange de pigments et peuvent ainsi se présenter sous forme de pigments nacrés ou interférentiels.

Les particules minérales colorées sont en particulier des particules non blanches, choisies de préférence parmi les oxydes de fer, à l'exclusion de l'oxyde de fer lamellaire, le bleu outremer (qui est un sulfosilicate complexe), les oxydes de chrome, le violet de manganèse (qui est un pyrophosphate d'ammonium et de manganèse) et le bleu de prusse (qui est un ferricyanure de fer).

De tels pigments mélaniques composites, déposés sur charge minérale colorée, sont décrits dans la demande de brevet français 92 0415 déposée le 16 Janvier 1992.

Les particules lamellaires sont des particules minérales ou organiques se présentant sous forme de feuillets éventuellement stratifiés. Ces feuillets se caractérisent par une épaisseur plus faible que la plus grande dimension de la particule. De préférence, le rapport entre la plus grande dimension et l'épaisseur est compris entre 2 et 100. La dimension la plus grande est généralement inférieure à 50 micromètres. De tels pigments mélaniques composites déposés sur charge lamellaire sont décrits, ainsi que leur préparation, dans la demande de brevet européen n°467.767

Les particules organiques non lamellaires sont des particules de polymères inertes choisis parmi les polymères naturels ou synthétiques, organiques ou inorganiques, à réseau réticulé, cristallin ou amorphe, ayant par exemple un poids moléculaire compris entre 5 000 et 5 000 000. Des pigments mélaniques composites sur charge polymérique ainsi que leur préparation sont décrits dans la demande de brevet européen n°379 409 ;
- les particules obtenues par polymérisation oxydative d'au moins un composé indolénique de formule : 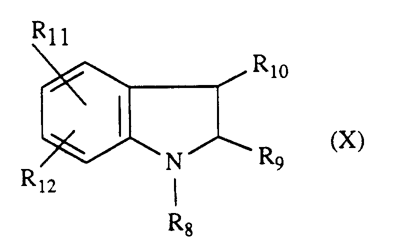 formule dans laquelle :
- R₁₀ et R₈ représentent, indépendamment l'un de l'autre, un atome d'hydrogène ou un radical alkyle en C₁-C₄ ;
- R₉ représente un atome d'hydrogène, un radical alkyle en C₁-C₄, un groupe carboxyle ou alcoxy (C₁-C₄) carbonyle;
- R₁₂ désigne un atome d'hydrogène, un radical alkyle en C₁-C₄, hydroxyle, alcoxy (C₁-C₄), amino ou alkylamino en C₁-C₁₀, ou halogène ;
- R₁₁ désigne un atome d'hydrogène, un groupe hydroxyle, alcoxy en C₁-C₄, ou amino ; avec la condition qu'au moins un des radicaux R₁₁ ou R₁₂ désigne un groupe hydroxyle,alcoxy ou amino; et avec la condition que, lorsque R₁₁ désigne un groupe amino, R₁₂ ne peut pas désigner un radical alkylamino ;
- R₁₁ et R₁₂ pouvant également former un groupement alkylènedioxy en C₁-C₂, lorsqu'ils sont en positions 5 et 6 ;
et leurs sels.

Les composés répondant à la formule (X) sont choisis notamment dans le groupe constitué par la 5,6-dihydroxyindoline, la 6-hydroxyindoline, la 5,6-méthylènedioxyindoline, la 7-méthoxy 6-hydroxyindoline, la 6,7-dihydroxyindoline, la 5-hydroxy 4-méthoxyindoline, la 4,5-dihydroxyindoline, la 5-méthoxy 6-hydroxyindoline, la 4-hydroxy 5-méthoxyindoline, la 5-hydroxy 6-méthoxyindoline, la 4,7-dihydroxyindoline, la 6-aminoindoline, la N-éthyl 4-hydroxyindoline, la 1-éthyl 6-aminoindoline, la 5,6-diaminoindoline, la 1-méthyl 6-aminoindoline, la 2-méthyl 6-aminoindoline, la 3-méthyl 6-aminoindoline, la 2-méthyl 5,6-diaminoindoline, la 5-chloro 7-aminoindoline, la 3-méthyl 5,7-diaminoindoline, la 5,7-diaminoindoline, la 2-méthyl 5,7-diaminoindoline, la 7-aminoindoline, la 2-méthyl 7-aminoindoline, la 4-aminoindoline, la 4-amino 6-chloroindoline, la 4-amino 6-iodoindoline, la 4-amino 5-bromoindoline, la 4-amino 5-hydroxyindoline, la 4-amino 7-hydroxyindoline, la 4-amino 5-méthoxyindoline, la 4-amino 7-méthoxyindoline, la 5-aminoindoline, la 2,3-diméthyl 5-aminoindoline, la 1-méthyl 5-aminoindoline, la 2-méthyl 5-aminoindoline, la 5-N-(1-méthylhexyl)aminoindoline, la 5,6-diméthoxyindoline et la 5,6-dihydroxy 2-carboxyindoline.

Dans les composés de formule (X), les radicaux alkyle en C₁-C₄ désignent de préférence méthyle, éthyle, propyle, isopropyle, butyle, isobutyle ; les radicaux en C₁-C₁₀ désignent de préférence méthyle, éthyle, propyle, isopropyle, butyle, isobutyle, 1-méthylhexyle, 1-méthylheptyle, 1-méthyloctyle ; les radicaux alcoxy désignent, de préférence, méthoxy, éthoxy, propoxy et butoxy; halogène désigne, de préférence, brome, chlore ou iode.

Les sels des composés de formule (X) sont, en particulier, des chlorhydrates, bromhydrates, sulfates, méthanesulfonates ou des sels de métaux alcalins, alcalino-terreux, d'ammonium ou d'amines.
- Les particules obtenues par co-oxydation d'au moins un composé indolinique de formule (X) et d'au moins un dérivé d'indole. Ce dernier peut être choisi parmi les mono- et di- hydroxyindoles ou les aminoindoles, tels que décrits plus particulièrement dans le brevet EP-A-2 398 826 et les demandes de brevet EP-A-425 345 et GB-A-2 224 754.

Ces indoles répondent plus particulièrement à la formule (IX). Lors de la co-oxydation, on peut utiliser jusqu'à 50% en mole de dérivés indoliques par rapport au nombre total de moles de dérivés à oxyder. Les conditions d'oxydation sont identiques à celles des pigments mélaniques décrites ci-dessus.

Tout comme les pigments mélaniques, les produits issus de la polymérisation oxydative d'au moins un composé indolinique de formule (X) peuvent être présents à la surface d'une charge particulaire, ou incorporés dans ladite charge particulaire minérale ou organique, lamellaire ou non lamellaire, colorée ou non. Ce sont alors des pigments composites.

Les charges particulaires minérales sont celles mentionnées ci-dessus pour les pigments mélaniques composites.

Les charges organiques non lamellaires sont choisies parmi les particules de :
a) polymères dérivés de la kératine, éventuellement modifiés;
b) fibroïnes de soie;
c) polymères dérivés de la chitine, éventuellement désacétylés ;
d) les polymères cellulosiques ;
e) polymères synthétiques choisis parmi :
   (i) le polyéthylène, le polypropylène, le polystyrène, le polyméthacrylate de méthyle éventuellement réticulé;
   (ii) la poly-β-alanine réticulée;
   (iii) les polymères réticulés de styrène/divinylbenzène, de méthacrylate de méthyle/diméthacrylate d'éthylèneglycol ou de stéarate de vinyle/divinylbenzène ;
   (iv) les microsphères creuses de copolymères de chlorure de vinylidène et d'acrylonitrile ;
   (v) des microsphères poreuses de polyamide 12, de polyamide 6 ou de copolyamide 6/12 ;
   (vi) de poudres de silicones constituées notamment par des gommes, des résines ou des élastomères d'organosiloxane.

De telles charges organiques non lamellaires sont mentionnées notamment dans la demande de brevet européen n°379 409.

Les charges lamellaires sont choisies parmi la L-lauroyllysine, les microparticules de céramique éventuellement recouvertes de poudre de zirconium, le dioxyde de titane lamellaire, le talc lamellaire, le nitrure de bore, le mica lamellaire, l'oxychlorure de bismuth, l'oxyde de fer transparent rouge.

De telles charges lamellaires sont notamment mentionnées dans la demande de brevet européen n°467 767.

De tels pigments composites ainsi que leur préparation sont notamment décrits dans la demande de brevet français n°92-00417 déposée le 16 Janvier 1992.

Dans les compositions de l'invention, les proportions des particules revêtues, dispersées dans le liant, dépendent du type de compositions ; il s'agit des proportions usuelles pour le type de composition considéré.

Pour enrober les particules, on peut utiliser une méthode connue, par exemple l'une des méthodes suivantes :
1) On prépare une solution du polymère dans un de ses bons solvants. On disperse dans cette solution la poudre à enrober sous agitation vigoureuse et on ajoute un mauvais solvant du polymère sans aller jusqu'à la précipitation du polymère dans la solution, mais jusqu'au premier trouble. On laisse alors la suspension sous agitation vigoureuse, par exemple pendant 4 heures. On laisse décanter la suspension, on rince avec un non-solvant du polymère et on sèche, par exemple à 80°C sous pression réduite.
2) On prépare une solution du polymère dans laquelle on disperse la poudre à enrober. On laisse le système sous agitation vigoureuse et on ajoute lentement un précipitant du polymère de façon à faire précipiter doucement le polymère à la surface de la poudre. On laisse décanter, on rince avec un non-solvant du polymère et on sèche la poudre.
3) On prépare une solution, avec un bon solvant du polymère, et on y disperse la poudre à enrober. On chosit un mauvais solvant du polymère dont le point d'ébullition est supérieur à celui du bon solvant et on réalise une évaporation lente du système. On aboutit à la formation d'un coacervat qui enrobe progressivement la poudre, puis on sèche la poudre.
4) On utilise la technique dite du lit d'air fluidisé ; on pulvérise, à chaud, une solution diluée du polymère dans un cyclone dans lequel la poudre est maintenue en sustentation.
5) On prépare une solution du polymère dans laquelle on disperse la poudre à enrober. On laisse le système sous agitation vigoureuse et on évapore lentement le solvant de façon à faire précipiter doucement le polymère à la surface de la poudre. On laisse décanter, on rince avec un non-solvant du polymère, et on sèche la poudre.
6) On utilise la technique d'enrobage par atomisation. On prépare une suspension de particules dans l'eau ; une fois que la suspension est homogénéisée, on y introduit une solution aqueuse du polymère. On laisse le mélange sous agitation pendant 2 heures et on atomise la suspension dans un appareil atomiseur. Durant l'atomisation, le mélange reste, de préférence, sous agitation magnétique.
7) On utilise la technique d'enrobage par lyophylisation. Pour cela, on solubilise le polymère dans l'eau, puis on y incorpore sous agitation magnétique une suspension aqueuse de particules. On laisse sous agitation magnétique pendant 6 à 8 heures, puis on met le mélange sur le lyophilisateur pendant au moins 18 heures. On récupère un produit pulvérulent que l'on tamise.

Dans les compositions de l'invention, le liant dans lequel sont dispersées les particules revêtues est un liant usuel. Les liants sont choisis par exemple parmi les corps gras (huiles et/ou cires), ou les polymères filmogènes.

Les compositions de l'invention peuvent être des compositions anhydres. Les compositions anhydres se présentent notamment sous la forme d'une poudre compactée, d'une poudre coulée, d'un rouge à lèvres ou d'un vernis à ongles.

Les compositions de l'invention peuvent également se présenter sous la forme d'émulsions du type eau-dans-l'huile ou huile-dans-l'eau.

Ces compositions sont préparées selon les méthodes usuelles.

Dans les compositions de maquillage, le liant est un liant gras usuel constitué d'une huile, d'un mélange d'huiles ou d'un mélange d'huile et de cire(s).

Dans les rouges à lèvres, le liant est également un liant gras généralement constitué par un mélange de cires de haut point de fusion (naturelles ou synthétiques), d'huiles (synthétiques, minérales ou végétales) et de cires à bas point de fusion (naturelles ou synthétiques).

Dans les vernis à ongles, le liant est constitué par la solution d'un polymère filmogène et d'un plastifiant dans le solvant organique choisi.

Dans les émulsions, le liant est un liant gras usuel constitué d'une huile ou d'un mélange d'huiles.

Lorsque la composition comporte un pigment micronisé d'oxydes métalliques choisis parmi les oxydes de titane, de zinc, de cérium, de zirconium ou leur mélanges, elle peut constituer une composition protectrice de la peau ou des cheveux contre les rayons ultra-violets.

L'invention concerne également l'utilisation, dans la préparation d'une composition cosmétique pour la peau et les phanères, contenant une dispersion de particules solides dans un liant, de particules dont la surface est revêtue avec au moins un polymère amphotère. Dans cette utilisation, la composition, et notamment les particules, le polymère amphotère ainsi que le liant, sont en particulier tels que définis ci-dessus.

Les exemples suivants illustrent l'invention.

### EXEMPLE 1

On introduit dans 200ml d'eau 0,5g d'un copolymère de méthacrylate de méthyle/méthacrylate d'éthyl diméthyl carboxyméthyl ammonium (Mexomer® PX de Chimex) et on attend sa dissolution complète.

Parallèlement, on disperse 50g de talc dans 550ml d'eau et une fois que la suspension est homogène, on ajoute la préparation précédente sous agitation.

On maintient l'agitation 24 heures. Le talc est ensuite récupéré par centrifugation, séché, broyé et tamisé sur tamis de mailles 0,160µm.

L'analyse élémentaire du résidu organique indique la présence de 0,92% de polymère déposé sur le talc (pourcentage en poids par rapport au talc).

Selon le même mode opératoire on a enrobé également des particules :
- d'oxyde de manganèse,
- de dioxyde de titane,
- d'oxyde de chrome.

### EXEMPLE 2

On introduit 7,15 g de copolymère d'acide acrylique et de chlorure de diméthyldiallylammonium (Merquat® 280 de MERCK) dans 200ml d'eau et on agite jusqu'à dissolution complète.

On y ajoute une suspension homogène de 50g de talc dans 550ml d'eau et on laisse sous agitation pendant 24 heures. On opère la séparation liquide/solide par centrifugation. Le culot est ensuite séché, broyé et tamisé.

Le talc obtenu présente un enrobage de 1,65% (% poids) de polymère amphotère.

### EXEMPLE 3

On introduit 2,25g d'octylacrylamide/acrylate/méthacylate de butylaminoéthyle (Amphomer® de National Starch) dans 200ml d'eau en présence de 0,0915 g d'aminométhyl propanol.

Une fois le polymère entièrement dissous, on y ajoute une suspension homogène de 50g de talc dans 550ml d'eau. On maintient le tout sous agitation pendant 4 heures.

On introduit ensuite dans le mélange un non-solvant, soit 10 ml d'une solution d'HCl 0,1M, et on poursuit l'agitation encore 20 heures. Le talc enrobé est séparé par centrifugation, séchage, broyage et tamisage. On récupère ainsi une poudre possédant en surface 0,68% de polymère (%poids).

De façon analogue, on a préparé un talc enrobé avec 1% du même polymère.

### EXEMPLE 4

On prépare une suspension de 10g de talc dans 100 ml d'eau. Une fois cette suspension homogénéisée, on y introduit une solution contenant 0,2g de copolymère méthacrylate de méthyle/méthacrylate d'éthyl diméthylcarboxyméthyl ammonium (Mexomere® PX de Chimex) dissous dans 50ml d'eau. On laisse sous agitation durant 12 heures.

On atomise cette suspension avec un atomiseur de laboratoire (Atomiseur Büchi 190 et Roucaire), dans les conditions suivantes :

| | |
|---|---|
| entrée | 135°C |
| sortie | 70°C |
| pompe | 7 bars |
| aspirateur | 7 bars |
| débit | 700 litres/heure |
| Durée de l'atomisation | 45 minutes |

On obtient une poudre sèche enrobée à 2,08% (% poids) de polymère.

La poudre obtenue est très légère et d'aspect cotonneux.

### EXEMPLE 5

On introduit 3,18g de copolymère méthacrylate de méthyle/méthacrylate d'éthyl diméthyl carboxyméthyl ammonium (Mexomère® PX de Chimex) dans 10ml d'eau.

Une fois le polymère dissous, on ajoute cette solution à une suspension de 10g de talc dans 40ml d'eau.

On laisse le tout sous agitation magnétique pendant 7 heures, puis on lyophilise pendant 18 heures.

Le produit récupéré est tamisé sur tamis de maille 0,160µm. On obtient une poudre très pulvérulente, enrobée, d'après les résultats d'analyse élémentaire, à 6,94% de polymères (% en poids).

### EXEMPLE 6 : Enrobage de particules par une silicone amphotère (Abil® B 9950)

On introduit 0,5 g d'Abil B 9950 (Coldschmidt) dans 200 ml d'eau et on attend sa dissolution complète. On procède ensuite conformément au mode opératoire décrit dans l'exemple 1.

### EXEMPLES DE COMPOSITIONS

Dans ces exemples :
Sinnowax AO® est la désignation commerciale d'un mélange d'alcool cérylstéarylique et d'alcool cétylstéarylique polyoxyéthyléné (Henkel)
Geleol® est la dénomination commerciale d'un mélange de mono- et di- stéarates de glycérol (Gattefosse)
Veegum R® est la dénomination commerciale d'un silicate de magnésium aluminium (Vanderbilt).
Oramix L30® est la dénomination commerciale du lauroylsarcosinate de sodium (Seppic).
Blanose® est la dénomination commerciale de la carboxyméthyl cellulose (sel de sodium), vendue par Aqualon
Mygliol® 812 est un mélange de triglycérides d'acides caprylique-caprique (Henkel)
Imwitor® 780K est un mélange de mono- et diglycérides d'acide isostéarique estérifiés par l'acide succinique (commercialisé par Huls-France).

### EXEMPLE C1 : Formulation d'un fard à paupières :

| | |
|---|---|
| Talc enrobé par l'amphomer® à 1% (ex. 3) | 22,9% |
| Mica | 22,0% |
| Oxychlorure de Bismuth | 8,0% |
| Dioxyde de titane | 2,0% |
| Stéarate de zinc | 3,0% |
| Nylon 12 | 20,0% |
| Oxydes de fer | 15,6% |
| Liant | 6,5% |

Le liant contient (% en poids) :
alcool oléique 11%
vaseline 11%
Huile de paraffine 67%
Myristate d'isopropyle 11%

Pour préparer ce fard à paupières, on opère de la façon suivante.

Les charges particulaires autres que les oxydes de fer sont homogénéisées à l'aide d'un agitateur-démotteur type Baker-Perkin. On ajoute alors les oxydes de fer puis le liant.

De la même façon, on a préparé des fards à paupières contenant un talc enrobè, respectivement, par :
- Mexomère® PX à 1%
- Merquat® 280 à 5%.

On a également préparé un fard témoin analogue mais contenant le talc non enrobé.

Sur ces fards à paupières, on a réalisé un test d'analyse sensorielle sur un jury de 10 personnes, avec évaluation des caractéristiques suivantes:
- adhérence du fard au moment de l'application sur la paupière,
- couvrance du fard immédiatement après le maquillage,
- tenue du fard quatre heures après maquillage. Il s'agit là d'évaluer la présence éventuelle et l'importance de stries sur les paupières, correspondant à la migration du produit vers des zones préférentielles de la paupière.

Les résultats du test sont les suivants :

Les formules utilisant le talc revêtu ont été jugées supérieures au témoin, sur les trois critères énoncés, par 7 personnes sur 10.

Il est à noter que les meilleurs résultats sur ces trois critères ont été obtenus par les polymères amphotères qui portent leurs charges négatives et positives sur deux substituants latéraux différents (ex : Amphomer® et Merquat® 280).

### EXEMPLE C2

Emulsion huile/eau dans laquelle on a introduit 5% de violet de manganèse enrobé par le Mexomer® PX.

Cet exemple démontre la facilité de dispersion du violet de manganèse enrobé par un polymère amphotère dans une émulsion.

### Formulation utilisée :

| **Phase grasse** | |
|---|---|
| Sinowax AO® | 7% |
| Geleol® | 2% |
| Alcool cétylique | 1,5% |
| Polydiméthylsiloxane | 1,5% |
| p-hydroxybenzoate de butyle | 0,2% |
| Huile de vaseline | 15% |

| **Pigment** | |
|---|---|
| Violet de manganèse enrobé par 1% de Mexomere® PX | 5% |

| **Phase aqueuse** | |
|---|---|
| Glycérine | 20% |
| Eau | 47,6% |
| Imidazolidinyl urée (conservateur) | 0,2% |

Le polydiméthylsiloxane est le composé vendu sous la dénomination Silbione® Oils 70047 V300 (Rhône-Poulenc)

L'émulsion est préparée selon un mode opératoire classique : les pigments sont introduits dans la phase aqueuse et on prépare l'émulsion en introduisant la phase grasse dans la phase aqueuse.

Par comparaison avec une émulsion témoin contenant le même pourcentage de violet de manganèse non enrobé, la mise en oeuvre de l'émulsion contenant le violet de manganèse enrobé est plus facile. Ce pigment se disperse mieux. L'observation au microscope de l'émulsion montre une dispersion du pigment enrobé plus homogène que dans le cas du même pigment non enrobé.

**EXEMPLE C3** : Fond de teint, contenant 10,7% de dioxyde de titane enrobé par par le MEXOMERE® PX.

### Formule utilisée :

| **PHASE A :** | |
|---|---|
| Eau | 30,34% |
| p-hydroxybenzoate de méthyle | 0,1% |
| Propylène glycol | 2% |
| Oxyde de fer noir | 0,5% |
| Dioxyde de titane enrobé à 1% de Méxomère PX | 10,2% |
| Veegum R | 1% + 10g d'eau |
| Blanose® | 0,16% + 10g d'eau |
| Oramix L30® | 0,6% |
| Triéthanolamine | 1% |

| **PHASE B :** | |
|---|---|
| Acide stéarique | 2,2% |
| Geleol® | 2,2% |
| Miglyol® 812 | 15% |
| p-hydroxybenzoate de propyle | 0,1% |
| Cyclopenta diméthylsiloxane (Dow Corning 24 S Fluid) | 12% |

| **PHASE C** | |
|---|---|
| Eau | 1% |
| Imidazolidinyl urée | 0,3% |
| Glycérine | 3% |

On prépare la phase A à chaud (80°C) sous agitation, on y introduit la phase B sous agitation et on y ajoute la phase C. On refroidit. On obtient un fond de teint rosé et fluide.

L'observation de cette émulsion au microscope comparée à la composition témoin (semblable mais contenant du dioxyde de titane non enrobé), montre une dispersion beaucoup plus régulière.

### EXEMPLE C4 :

Formulation d'un fond de teint siliconé dont tous les pigments sont enrobés par une silicone amphotère : l'Abil® B 9950 du Goldschmidt.

| | |
|---|---|
| Paillettes d'aluminium recouvertes d'un vernis époxy coloré (Kingston Avocado MI.Synthecolor) | 5% |
| Cyclopenta diméthylsiloxane (volatil Silicone 7158, Union Carbide) | 15 % |
| Myglyol® 812 | 4% |
| Imwitor® 780K | 2% |
| Oxyde de fer jaune enrobé par 1% d'Abil® B 9950 | 1,43% |
| Oxyde de fer rouge enrobé par 0,5% d'Abil B 9950 | 0,55% |
| Oxyde de fer noir enrobé par 1% d'Abil® B 9950 | 0,23% |
| Dioxyde de titane enrobé par 0,5% % d'Abil® B 9950 | 4,80% |
| Eau | 34,50% |
| Blanose® 7 LF (Aqualon) | 0,50% |
| Glycérine | 15% |
| p-Hydroxybenzoate de méthyle | 0,2% |
| Eau | 6% |
| Eau | 5,8% |
| Sulfate de magnésium hydraté à 7H₂O | 0,7% |
| Eau | 7% |
| Imidazolidinylurée (Biopur® 100, Biophil) | 0,3% |
| Mélange de polydiméthylsiloxane-ol et de cyclopentadiméthyl-siloxane, Dow Corning QC F2-1671 (Dow Corning) | 4% |

En comparaison avec la même formule contenant les pigments enrobés par 5% de PDMS (polydiméthylsiloxane commercialisé par Wackherr), le fond de teint ci-dessus présente une texture plus onctueuse et lisse, une couleur plus intense. La dispersion des pigments est bonne, l'émulsion est fine, régulière, et stable après centrifugation.Le maquillage obtenu est très homogène.

## Revendications

1. Composition cosmétique pour la peau ou les phanères, comprenant une dispersion de particules solides dans un liant, caractérisée par le fait qu'au moins une partie desdites particules sont introduites dans ladite composition sous la forme de particules dont la surface est revêtue d'au moins un polymère amphotère, ledit polymère amphotère contenant à la fois des groupements cationiques et anioniques et/ou des groupements ionisables respectivement en groupements cationiques et anioniques.

2. Composition selon la revendication 1, caractérisée par le fait que ledit polymère amphotère ne contient pas de silicium.

3. Composition selon l'une quelconque des revendications précédentes, caractérisée par le fait que lesdits groupements cationiques, ou ionisables en groupements cationiques, sont choisis parmi ceux qui contiennent des groupements amines primaires, secondaires, tertiaires, et/ou quaternaires.

4. Composition selon l'une quelconque des revendications précédentes caractérisée par le fait que lesdits groupements cationiques ou ionisables en groupements cationiques sont portés par un substituant latéral de la chaîne polymère.

5. Composition selon l'une quelconque des revendications précédentes, caractérisée par le fait que lesdits groupements anioniques sont choisis parmi les groupements COO⁻, SO₃⁻, PO₄³⁻, SO₄²⁻.

6. Composition selon l'une quelconque des revendications 1 à 5, caractérisée par le fait que lesdits groupements anioniques sont portés par un substituant latéral.

7. Composition selon l'une quelconque des revendications 1 à 6, caractérisée par le fait que lesdits groupements anioniques et cationiques sont portés chacun par un substituant latéral.

8. Composition selon l'une quelconque des revendications 1 à 7, caractérisée par le fait que le rapport groupements cationiques/groupements anioniques s'échelonne de 40/60 à 90/10.

9. Composition selon l'une quelconque des revendications précédentes, caractérisée par le fait que la proportion pondérale de polymères amphotères, dans les particules revêtues, est inférieure à 10%, par rapport au poids total des particules revêtues, et de préférence inférieure à 8%.

10. Composition selon la revendication précédente, caractérisée par le fait que ladite proportion ne dépasse pas 2% en poids.

11. Composition selon l'une quelconque des revendications précédentes, caractérisée par le fait que lesdites particules revêtues comprennent des charges ou des pigments minéraux.

12. Composition selon l'une quelconque des revendications précédentes, caractérisée par le fait que lesdites particules revêtues comprennent des pigments organiques ou des pigments composites à revêtement organique.

13. Composition selon l'une quelconque des revendications précédentes, caractérisée par le fait que le liant est choisi parmi les corps gras (huiles et/ou cires) et les polymères filmogènes.

14. Composition selon l'une quelconque des revendications précédentes, caractérisée par le fait qu'elle se présente sous la forme d'une poudre compactée, d'une poudre coulée, d'un rouge à lèvres, ou d'un vernis à ongles.

15. Composition selon l'une quelconque des revendications 1 à 13, caractérisée par le fait qu'elle se présente sous la forme d'une émulsion du type eau-dans-l'huile ou huile-dans-l'eau.

16. Utilisation, dans la préparation d'une composition cosmétique pour la peau ou les phanères contenant une dispersion de particules solides dans un liant, de particules dont la surface est revêtue avec au moins un polymère amphotère, ledit polymère amphotère contenant à la fois des groupements cationiques et anioniques et/ou des groupements ionisables respectivement en groupements cationiques et en groupements anioniques.

17. Utilisation selon la revendication précédente, caractérisée par le fait que ladite composition cosmêtique est telle que définie dans l'une quelconque des revendications 1 à 10.

## Claims

1. Cosmetic composition for the skin or the exoskeleton comprising a dispersion of solid particles in a binding agent, characterized in that at least a part of the said particles is introduced into the said composition in the form of particles whose surface is coated with at least one amphoteric polymer, the said amphoteric polymer containing both cationic and anionic groups and/or groups which can ionize to cationic and anionic groups, respectively.

2. Composition according to Claim 1, characterized in that the said amphoteric polymer does not contain silicon.

3. Composition according to either of the preceding claims, characterized in that the said cationic groups, or groups which can ionize to cationic groups, are chosen from those which contain primary, secondary, tertiary and/or quaternary amine groups.

4. Composition according to any one of the preceding claims, characterized in that the said cationic groups, or groups which can ionize to cationic groups, are carried by a side-chain substituent of the polymer chain.

5. Composition according to any one of the preceding claims, characterized in that the said anionic groups are chosen from COO⁻, SO₃⁻, PO₄³⁻ and SO₄²⁻ groups.

6. Composition according to any one of Claims 1 to 5, characterized in that the said anionic groups are carried by a side-chain substituent.

7. Composition according to any one of Claims 1 to 6, characterized in that the said anionic and cationic groups are each carried by a side-chain substituent.

8. Composition according to any one of Claims 1 to 7, characterized in that the ratio of cationic groups to anionic groups ranges from 40:60 to 90:10.

9. Composition according to any one of the preceding claims, characterized in that the weight proportion of amphoteric polymers in the coated particles is less than 10% relative to the total weight of the coated particles, and preferably less than 8%.

10. Composition according to the preceding claim, characterized in that the said proportion does not exceed 2% by weight.

11. Composition according to any one of the preceding claims, characterized in that the said coated particles comprise inorganic fillers or pigments.

12. Composition according to any one of the preceding claims, characterized in that the said coated particles comprise organic pigments or composite pigments with an organic coating.

13. Composition according to any one of the preceding claims, characterized in that the binding agent is chosen from fats (oils and/or waxes) and film-forming polymers.

14. Composition according to any one of the preceding claims, characterized in that it takes the form of a compact powder, a loose powder, a lipstick or a nail varnish.

15. Composition according to any one of Claims 1 to 13, characterized in that it takes the form of a water-in-oil or oil-in-water type emulsion.

16. Use, in the preparation of a cosmetic composition for the skin or the exoskeleton containing a dispersion of solid particles in a binding agent, of particles whose surface is coated with at least one amphoteric polymer, the said amphoteric polymer containing both cationic and anionic groups and/or groups which can ionize to cationic groups and to anionic groups, respectively.

17. Use according to the preceding claim, characterized in that the said cosmetic composition is as defined in any one of Claims 1 to 10.

## Patentansprüche

1. Kosmetische Zubereitung für die Haut oder deren Anhanggebilde mit einem Gehalt an einer Dispersion fester Partikel in einem Bindemittel, dadurch gekennzeichnet, daß mindestens ein Teil dieser Partikel in diese Zubereitung in Form von Partikeln eingearbeitet ist, deren Oberfläche mit mindestens einem amphoteren Polymer überzogen ist, wobei das amphotere Polymer gleichzeitig kationische und anionische Gruppierungen und/oder jeweils an den kationischen und anionischen Gruppen ionisierbare Gruppierungen aufweist.

2. Zubereitung nach Anspruch 1, dadurch gekennzeichnet, daß das amphotere Polymer kein Silizium enthält.

3. Zubereitung nach einem der vorstehenden Ansprüche, dadurch gekennzeichnet, daß die kationischen oder an den kationischen Gruppen ionisierbaren Gruppierungen aus solchen ausgewählt sind, die primäre, sekundäre, tertiäre und/oder quaternäre Gruppen enthalten.

4. Zubereitung nach einem der vorstehenden Ansprüche, dadurch gekennzeichnet, daß die kationischen oder an den kationischen Gruppen ionisierbaren Gruppierungen von einem seitlichen Substituenten der Polymerkette getragen werden.

5. Zubereitung nach einem der vorstehenden Ansprüche, dadurch gekennzeichnet, daß die anionischen Gruppierungen aus den Gruppen COO⁻, SO₃⁻, PO₄³⁻ oder SO₄²⁻ ausgewählt sind.

6. Zubereitung nach einem der Ansprüche 1 bis 5, dadurch gekennzeichnet, daß die anionischen Gruppierungen von einem seitlichen Substituenten getragen werden.

7. Zubereitung nach einem der Ansprüche 1 bis 6, dadurch gekennzeichnet, daß die anionischen und kationischen Gruppierungen jeweils von einem seitlichen Substituenten getragen werden.

8. Zubereitung nach einem der Ansprüche 1 bis 7, dadurch gekennzeichnet, daß das Verhältnis der kationischen zu den anionischen Gruppierungen im Bereich von 40/60 bis 90/10 eingestuft ist.

9. Zubereitung nach einem der vorstehenden Ansprüche, dadurch gekennzeichnet, daß das Gewichtsverhältnis an amphoteren Polymeren bei den überzogenen Teilchen weniger als 10 Gew.-% in bezug auf das Gesamtgewicht der überzogenen Partikel, vorzugsweise weniger als 8 Gew.-%, beträgt.

10. Zubereitung nach dem vorstehenden Anspruch, dadurch gekennzeichnet, daß das Verhältnis 2 Gew.-% nicht überschreitet.

11. Zubereitung nach einem der vorstehenden Ansprüche, dadurch gekennzeichnet, daß die überzogenen Partikel Trägerstoffe oder mineralische Pigmente umfassen.

12. Zubereitung nach einem der vorstehenden Ansprüche, dadurch gekennzeichnet, daß die überzogenen Partikel organische Pigmente oder zusammengesetzte Pigmente mit organischem Überzug umfassen.

13. Zubereitung nach einem der vorstehenden Ansprüche, dadurch gekennzeichnet, daß das Bindemittel aus Fettkörpern (Ölen und/oder Wachsen) und filmbildenden Polymeren ausgewählt ist.

14. Zubereitung nach einem der vorstehenden Ansprüche, dadurch gekennzeichnet, daß sie in Form eines Kompaktpuders, eines freifließenden Puders, als Lippenstift (Lippenrouge) oder als Nagellack vorliegt.

15. Zubereitung nach einem der Ansprüche 1 bis 13, dadurch gekennzeichnet, daß sie in Form einer Emulsion vom Typ Wasser-in-Öl oder Öl-in-Wasser vorliegt.

16. Verwendung von Partikeln, deren Oberfläche mit mindestens einem amphoteren Polymer überzogen ist bei, der Herstellung einer kosmetischen Zubereitung für die Haut oder deren Anhanggebilde, wobei dieses amphotere Polymer gleichzeitig kationische und anionische Gruppierungen und/oder jeweils an den kationischen und anionischen Gruppen ionisierbare Gruppierungen enthält.

17. Verwendung nach dem vorstehenden Anspruch, dadurch gekennzeichnet, daß die kosmetische Zubereitung die in einem der Ansprüche 1 bis 10 definierte Zusammensetzung aufweist.
